# EUROPEAN PATENT SPECIFICATION

(11) **EP 1 653 937 B1**
(45) Date of publication and mention of the grant of the patent: **06.01.2010**
(21) Application number: 04745029.1
(22) Date of filing: 28.07.2004
(51) Int. Cl.: A61K 31/16, A61K 31/19

(54) **COMPOUNDS USEFUL FOR TREATING NEUROPATHIC PAIN AND MIGRAINE**
VERBINDUNGEN ZUR BEHANDLUNG VON NEUROPATHISCHEN SCHMERZEN UND MIGRÄNE
COMPOSES UTILISES POUR TRAITER DE LA DOULEUR NEUROPATHIQUE ET DE LA MIGRAINE

(30) Priority: 28.07.2003 US 490273 P
(43) Date of publication of application: 10.05.2006
(73) Proprietor: YISSUM RESEARCH DEVELOPMENT COMPANY OF THE HEBREW UNIVERSITY OF JERUSALEM, Givat Ram, Jerusalem 91390 (IL)
(72) Inventor: BIALER, Meir, 96222 Jerusalem (IL); YAGEN, Boris, 93707 Jerusalem (IL); WINKLER, Ilan, 59475 Bat Yam (IL); DEVOR, Marshall, 95743 Jerusalem (IL)
(74) Representative: Grünecker, Kinkeldey, Stockmair & Schwanhäusser Anwaltssozietät
(86) International application number: PCT/IL2004/000689
(87) International publication number: WO 2005/009430

(56) References cited:
- US-B1- 6 417 399
- ISOHERRANEN N ET AL: "NEW CNS-ACTIVE DRUGS WHICH ARE SECOND-GENERATION VALPROIC ACID: CAN THEY LEAD TO THE DEVELOPMENT OF A MAGIC BULLET?" CURRENT OPINION IN NEUROLOGY, RAPID SCIENCE PUBLISHERS, LONDON, GB, vol. 16, no. 2, April 2003 (2003-04), pages 203-211, XP008035486 ISSN: 1350-7540
- ISOHERRANEN, N. ET AL.: "Anticonvulsant Profile and Teratogenicity of N-methyl-tetramethylcyclopropyl Carboxamide: A New Antiepileptic Drug" EPILEPSIA, vol. 43, no. 2, 25 October 2001 (2001-10-25), pages 115-126, XP002302527

## Description

The invention relates to compounds for the treatment of neuropathic pain and, migraine.

Neuropathic pain is an intractable pain initiated or caused by a primary lesion or dysfunction in the peripheral or central nervous system. Neuropathic pain is part of the neurological disease spectrum and may be an expression of severe medical pathology [Hansson P. European J. of Pain 2002; 6; 45]. Neuropathic pain manifests itself due to neurological disorders accompanying various causes such as "wound, infection, cancer, ischemia and metabolic disorders including diabetes mellitus. Though there are many unclear points on the mechanism of neuropathic pain, it is considered that abnormal continuous firing of sensory nerve and the like are the cause. Typical symptoms of neuropathic pain include allodinia, hyperalgesia, hyperesthesia and the like. Their symptoms include characteristic pains expressed as "like burning", "like stinging", "like electrical shock" and the like. Unfortunately, and unlike other types of pain, neuropathic-pain tends to respond poorly to analgesic medication.

It is known that analgesics, particularly narcotic analgesics and the like, which are effective for general nociceptive pains are hardly effective for neuropathic pain. For example, it is known that morphine has a strong analgesic effect on nociceptive pains but does not show a sufficient effect on neuropathic pain.

Patients with neuropathic pain do not respond to non-steroidal anti-inflamatory drugs and resistence or insensitivity to opiates is common. Patients are usually treated empirically with tricyclic or serotonin and norepinephrine uptake inhibitors, and anticonvalsants that all have limited efficacy and undesirable side effects. Neurosurgical lesions have a negligible role and functional neurosurgery, including dorsal column or brain stimulation, is controversial, although transcutaneous nerve stimulation may provide some relief. Local anaesthetic blocks targeted at trigger points, peripheral nerves, plexi, dorsal roots, and the sympathetic nervous system have useful but short lived effects; longer lasting blocks by phenol injection or cryotherapy risk irreversible functional impairment and have not been tested in placebo-controlled trials. Chronic epidural administration of drugs such as clonidine, steroids, opiods, or midazolam is invasive, has side effects and the efficacy of these drugs has not been adequately assessed [Woolf J. et al. Lancet 1999; 353; 1959-64].

Valproic acid (VPA), is one of the major antiepileptic drugs used today, having a wide use in both generalized and partial epilepsies. VPA has not been well studied for effect on neuropathic pain and the role of VPA in the treatment of neuropathic pain has not been determined by clinical trials. In one double-blind placebo-controlled trial of VPA reported on so far for the treatment of neuropathic pain due to spinal cord injury there was no difference between VPA and placebo in relieving pain [Backonja M.M. The Clinical Journal of Pain, 16, S67-S72, 2000].

Additionally, the use of Valproic acid (VPA), is limited by its considerable adverse effects including hepatotoxicity and teratogenicity and thus cannot be given to women of childbearing age and children [Baille, T. A. et al. In Antiepileptic Drugs, eds. R.H. Levy et al. Raven Press, New York. Pp. 641-651 (1989)].

Since a safe and effective therapeutic method has not been established, concern has been directed toward the development of a therapy effective for neuropathic pain.

There is thus a widely recognized need for, and it would be highly advantageous to have, new agents for treating neuropathic pain devoid of the above limitations.

Valnoctamide (VCD), an amide analogue of VPA having anticonvulsant activity was found to be distinctly less teratogenic than VPA [Radatz M et al. Epilepsy Res. 1998:30(1):41-8]. M. Roeder et al [M. Roeder et al, Tetrahedron: Asymmetry 1999: 10: 841-853] and US Patent No. 6,417,399 relate to stereoisomers of valnoctamide (VCD), synthesis thereof, a method for stereoselective separation thereof as well as uses thereof.

U.S. patent No. 5,880,157 and M. Bialer et al. [M. Bialer et al. Pharm Res. 13:284-289 (1996)] disclose derivatives of 2,2,3,3 tetramethylcyclopropane carboxylic acid for treating epilepsy. Isoherranen N. et al 2002, studied the anticonvulsant activity of N-methyl-tetramethylcyclopropyl carboxamide (M-TMCD) and its metabolite in various animal (rodent) models of human epilepsy, and evaluated their ability to induce neural tube defects (NTDs) and neurotoxicity. [Isoherranen N. et al. Epilepsia 2002; 43:115 - 126]. M-TMCD (a cyclopropyl analog of VPA) was found to be advantageous compared to VPA because of its better potency as an anticonvulsant drug, its wider safety of margin, its lack of teratogenicity and its potential lack of hepatotoxicity.

Isoherranen et al. report in Current Opinion in Neurology 2003, 16: 203-211 about new CNS-active drugs which are second-generation valproic acid such as amide derviatives of valproic acid.

Thus, mere is a widely recognized need and it will be highly advantageous to have compounds which are effective in treating neuropathic pain with minimal side effects. Additionally, it would be highly advantageous to have compounds effective against migraine.

According one aspect of the present invention there is provided use of a compound of formula I 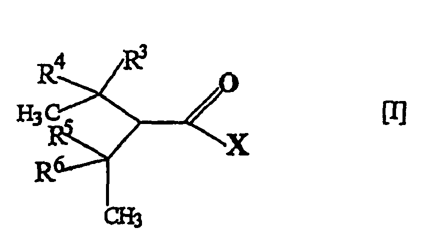 wherein
(i) X is selected from OH and NR¹R²;
(ii) R¹, R² are independently selected from H and C₁-C₆ alkyl; and
(iii)
   (a) R³, R⁴ are independently selected from H and C₁-C₆ alkyl; R⁵, R⁶ are independently selected from H and C₁-C₆ alkyl; or
   (b) one of R³ and R⁴, together with one of R⁵ and R⁶, form a cyclopropyl ring, and the other of R³, R⁴, R⁵ and R⁶ is selected from H and C₁-C₆ alkyl.
for the preparation of a medicament for treating a disease or condition selected from: neuropathic pain and migraine.

According to another aspect of the present invention there is provided a pharmaceutical composition for treating a disease or condition selected from: neuropathic pain and migraine, comprising a pharmaceutically acceptable carrier and as an active ingredient a therapeutically effective amount of a compound of formula I 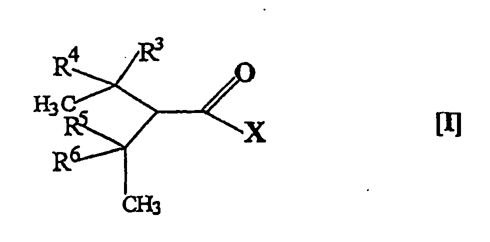 wherein
(i) X is selected from OH and NR¹R²;
(ii) R¹, R² are independently selected from H and C₁-C₆ alkyl; and
(iii)
   (a) R³, R⁴ are independently selected from H and C₁-C₆ alkyl;
      R⁵, R⁶ are independently selected from H and C₁-C₆ alkyl; or
   (b) one of R³ and R⁴, together with one of R⁵ and R⁶, form a cyclopropyl ring, and the other of R³, R⁴, R⁵ and R⁶ is selected from H and C₁-C₆ alkyl.

According to yet another aspect of the present invention there is provided a method for treating a disease or condition selected from: neuropathic pain and migraine, in a mammal comprising administering to the mammal, a therapeutically effective amount of a compound of formula I 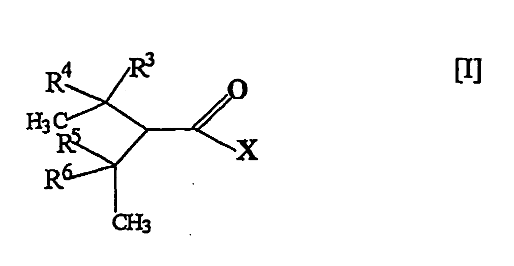 wherein
(i) X is selected from OH and NR¹R²;
(ii) R¹ R² are independently selected from H and C₁-C₆ alkyl; and
(iii)
   (a) R³, R⁴ are independently selected from H and C₁-C₆ alkyl;
      R⁵, R⁶ are independently selected from H and C₁-C₆ alkyl; or
   (b) one of R³ and R⁴, together with one of R⁵ and R⁶, form a cyclopropyl ring, and the other of R³, R⁴, R⁵ and R⁶ is selected from H and C₁-C₆ alkyl.

The present invention relates to the use of a compound of formula I

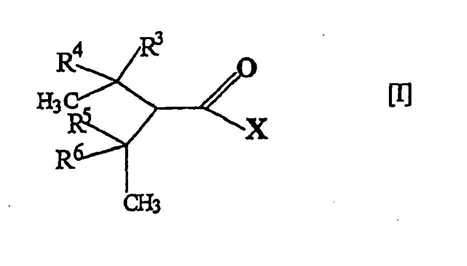

wherein
(i) X is selected from OH and NR¹R²;
(ii) R¹, R² are independently selected from H and C₁-C₆ alkyl; and
(iii)
   (a) R³, R⁴ are independently selected from H and C₁-C₆ alkyl;
      R⁵, R⁶ are independently selected from H and C₁-C₆ alkyl; or
   (b) one of R³ and R⁴, together with one of R⁵ and R⁶, form a cyclopropyl ring, and the other of R³, R⁴, R⁵ and R⁶ is selected from H and C₁-C₆ alkyl;
for the preparation of a medicament for treating a disease or condition selected from: neuropathic pain and migraine.

The present invention also includes pharmaceutical acceptably salts of the compounds of formula I.

It should be noted that the present invention excludes the compound valproic acid.

As used herein the term *"the other of R³, R⁴, R⁵ and R⁶"* refers to the two groups of R³, R⁴, R⁵ and R⁶ which do not form a cyclopropyl ring. For example, when R³ and R⁵ form together a cyclopropyl ring "the other..." is R⁴ and R⁶. When R⁴ and R⁵ form together a cyclopropyl ring "the other... " is R³ and R⁶, etc.

As used herein the term *"treating"* includes prophylactic and/or therapeutic uses and refers to abrogating, preventing, alleviating, slowing or reversing the progression of a disease or condition, or substantially preventing the appearance of clinical symptoms of a disease or condition.

As used herein the term *"neuropathic pain"* refers to any pain which initial cause was due to damage, or injury to the neural tissue. The predominant mechanism is aberrant somatosensory processing.

As used herein the term *"migraine"* refers to an often familial symptom complex of periodic attacks of vascular headache, usually temporal and unilateral in onset, commonly associated with irritability, nausea, vomiting, constipation or diarrhoea and often photophobia, attacks are preceded by constriction of the cranial arteries, usually with resultant prodromal sensory (especially ocular) symptoms and commence with the vasodilation that follows.

As used herein the term *"therapeutically effective amount"* " refers to an amount of a compound sufficient to prevent, inhibit, reduce, or eliminate one or more causes, symptoms, or complications of neuropathic pain and migraine.

The term "therapeutically effective amount" also refers to an amount of a compound sufficient to bring about at least one of the effects defined under the term treating.

Preferably the C₁-C₆ alkyl of R¹ and R² consists of 1-3 carbon atoms and most preferably the alkyl is a methyl.

Preferably the C₁-C₆ alkyl of R³, R⁴, R⁵ and R⁶-consists of 1-3 carbon atoms, more preferably 1-2 carbon atoms and most preferably the alkyl is a methyl.

As used herein the term "C₁-C₆allcyl" refers to a saturated aliphatic hydrocarbon of 1 to 6 carbon atoms. In case the alkyl includes 2-6 carbon atoms, the alkyl may have one or more carbon-carbon double bonds (termed also alkenyl). Thus, R³, R⁴, R⁵ and R⁶ may also be an alkenyl (an unsaturated straight or branched aliphatic hydrocarbon, having one or more carbon-carbon double bonds) including 2 to 6 carbon atoms. Examples of alkenyl groups include, without limitation, ethenyl, n-propenyl, isopropenyl etc.

Whenever a numerical range e.g. "1-6" is stated herein, it means that the group in this case the alkyl group, may contain 1 carbon atom, 2 carbon atoms, 3 carbon atoms, etc., up to and including 6 carbon atoms. The C₁-C₆alkyl group may be for example methyl, ethyl, propyl, isopropyl, butyl, isobutyl, tertiary butyl, sec-butyl, amyl, pentyl, isopentyl, or hexyl.

The C₁-C₆ alkyl group may be a straight or a branched alkyl group.

Preferably the total number of carbon atoms of R³, R⁴, R⁵ and R⁶ in a compound of formula I is two. The term *"the total number of carbon atoms of R³, R⁴, R⁵ and R⁶"* means the sum of carbon atoms of R³, R⁴, R⁵ and R⁶.

Preferably the total number of carbon atoms in a compound of formula I excluding the carbon atoms of R¹ and R² is eight.

According to a preferred embodiment of the present invention,
(i) X is OH; and
(ii)
   (a) R³, R⁴ are independently selected from H and C₁-C₆ alkyl;
      R⁵, R⁶ are independently selected from H and C₁-C₆ alkyl; or
   (b) one of R³ and R⁴, together with one of R⁵ and R⁶, form a cyclopropyl ring, and the other of R³, R⁴, R⁵ and R⁶ is selected from H and C₁-C₆ alkyl.

According to another preferred embodiment of the present invention,
(i) X is NR¹R²;
(ii) R¹ R² are independently selected from H and C₁-C₆ alkyl; and
(iii)
   (a) R³, R⁴ are independently selected from H and C₁-C₆ alkyl;
      R⁵, R⁶ are independently selected from H and C₁-C₆ alkyl; or
   (b) one of R³ and R⁴, together with one of R⁵ and R⁶, form a cyclopropyl ring, and the other of R³, R⁴, R⁵ and R⁶ is selected from H and C₁-C₆ alkyl.

Additionally according to a preferred embodiment of the present invention,
(i) X is selected from OH and NR¹R²;
(ii) R¹, R² are independently selected from H and C₁-C₆ alkyl; and
(iii)
   (a) R³, R⁴ are independently selected from H, methyl and ethyl;
      R⁵, R⁶ are independently selected from H, methyl and ethyl; or
   (b) one of R³ and R⁴, together with one of R⁵ and R⁶, form a cyclopropyl ring, and the other of R³, R⁴, R⁵ and R⁶ is selected from H, methyl and ethyl.

Moreover according to a more preferred embodiment of the present invention,
X is selected from OH and NR¹R²; R¹ R² are independently selected from H and C₁-C₆ alkyl; and one of R³ and R⁴, together with one of R⁵ and R⁶, form a cyclopropyl ring, and the other of R³, R⁴, R⁵ and R⁶ is a methyl.

Further according to a more preferred embodiment the compound of formula I is of structural formula II 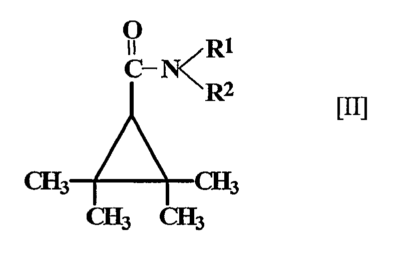 wherein R¹ and R² are independently selected from H and C₁-C₆ alkyl.

According to this preferred embodiment X is NR¹R²; R¹, R² are independently selected from H and C₁-C₆ alkyl; and one of R³ and R⁴, together with one of R⁵ and R⁶, form a cyclopropyl ring, and the other of R³, R⁴, R⁵ and R⁶ is a methyl.

Preferably the C₁-C₆ alkyl is a C₁-C₃ alkyl and most preferably a methyl.

Preferably at least one of R¹ and R² is H and the other of R¹ and R² is a C₁-C₆ alkyl.

As used herein the term *"the other of R¹ and R²"* refers to the non-hydrogen group. For example, if R¹ is H, then R² is C₁-C₆ alkyl and vice versa.

Preferably the C₁-C₆ alkyl of R¹ and R² is a C₁-C₃ alkyl and most preferably a methyl.

According to a preferred embodiment of the present invention one of R¹ and R² is H and the other of R¹ and R² is C₁-C₆ alkyl, more preferably C₁-C₃ alkyl and most preferably methyl.

According to another preferred embodiment of the present invention R¹ and R² are H.

Preferred compounds are
N-methyl-2,2,3,3-tetramethylcyclopropanecarboxamide (M-TMCD) - compound III;
2,2,3,3-tetramethylcyclopropanecarboxamide (TMCD)- compound IV; and 2-ethyl-3-methyl-pentanoic acid amide (Valnoctamide)- compound V.

More preferred compounds are N-methyl-2,2,3,3-tetramethylcyclopropanecarboxamide (M-TMCD) and 2-ethyl-3-methyl-pentanoic acid amide (Valnoctamide) and most preferred compound is N-methyl-2,2,3,3-tetramethylcyclopropanecarboxamide (M-TMCD).

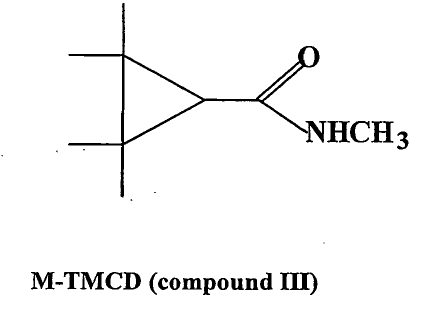

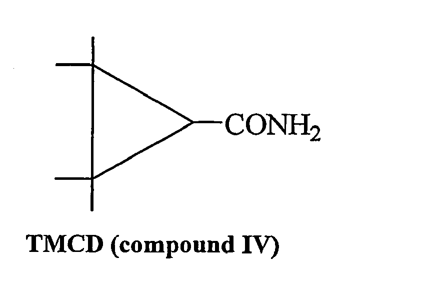

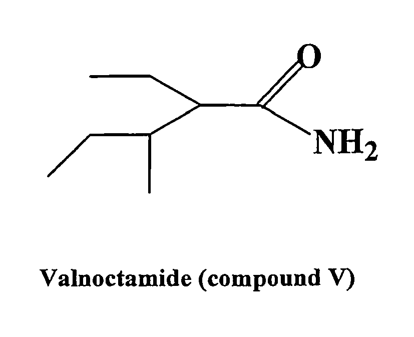

For purposes of this specification, the term VCD refers to 2-ethyl-3-methyl-pentanoic acid amide (Valnoctamide).

Additional preferred compounds are:
2-ethyl-3-methyl-pentanoic acid (valnoctic acid),
2,2,3,3-tetramethylcyclopropanecarboxylic acid (TMCA).

When X is OH; and one of R³ and R⁴, together with one of R⁵ and R⁶, form a cyclopropyl ring, and the other of R³, R⁴, and R⁶ is a methyl, the compound is TMCA.

According to a preferred embodiment of the present invention the disease or condition is a neuropathic pain.

Preferably the compound is administered as a pharmaceutical composition comprising a compound of formula I and a pharmaceutical acceptable carrier.

According to a preferred embodiment of the present invention the route of administration of the medicament is selected from oral, parenteral, topical, transdermal, mucosal, rectal and buccal administration.

According to a preferred embodiment of the present invention the route of administration of the compound is selected from oral, parenteral, topical, transdermal, mucosal, rectal and buccal administration.

More preferably the route of administration of the medicament is selected from oral and parenteral administration and most preferably oral administration.

More preferably the route of administration of the compound is selected from oral and parenteral administration and most preferably oral administration.

Preferably the parenteral route of administration is selected from intravenous, intramuscular, intraperitoneal and subcutaneous administration.
Preferably the mammal is a human.

The invention further relates to a pharmaceutical composition for treating a disease or condition selected from: neuropathic pain and migraine, comprising a pharmaceutically acceptable carrier and as an active ingredient a therapeutically effective amount of a compound of formula I 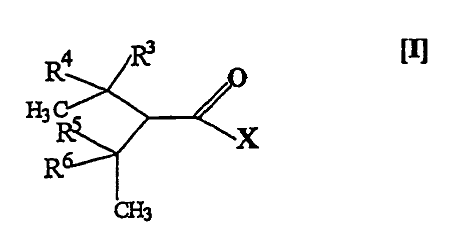 wherein
(i) X is selected from OH and NR¹R²;
(ii) R¹, R² are independently selected from H and C₁-C₆ alkyl; and
(iii)
   (a) R³, R⁴ are independently selected from H and C₁-C₆ alkyl;
      R⁵, R⁶ are independently selected from H and C₁-C₆ alkyl; or
   (b) one of R³ and R⁴, together with one of R⁵ and R⁶, form a cyclopropyl ring, and the other of R³, R⁴, R⁵ and R⁶ is selected from H and C₁-C₆ alkyl.

The pharmaceutical compositions of the present invention comprises as an active ingredient a therapeutically effective amount of at least one compound as described in the present invention and a pharmaceutically acceptable carrier.

As used herein a *"pharmaceutical composition"* refers to a preparation of one or more compounds described herein, with other inert chemical components such as suitable pharmaceutically acceptable carriers. The purpose of a pharmaceutical composition is to facilitate administration of a compound to a subject (mammal).

As used herein the term *"pharmaceutically acceptable carrier"* refers to an inert non-toxic carrier or diluent that does not cause significant irritation to a subject (mammal) and does not abrogate the biological activity and properties of the administered compound.
Examples without limitation of carriers are lactose, sucrose, water, organic solvents and polyethyleneglycol.

The carriers may include additional excipients such as binders, disintegrants, lubricants, surface active agents, preservatives and favoring agents.

The treatment may be prophylactic, for preventing the disease from occurring such as for preventing neuropathic pain following surgery by administration of the compound of the invention prior to surgery. Alternatively the administration may be performed after the disease or condition were already established so as to eliminate or decrease at least one of the manifestations of the disease or condition.

Preferably the disease is neuropathic pain, and the compound may be administered to prevent the occurrence of the pain (for example before amputation surgery, to a diabetic patient likely to develop diabetic neuropathy) or may be administered after the neuropathic pain is already established so as to eliminate or reduce the intensity of the pain as compared to the non-treated condition.

### Modes of administration and amounts

The method of administration of the compounds above may be oral, parenteral, topical, transdermal, mucosal or buccal. The pharmaceutical composition may also be administered rectally for example through the use of an enema or suppository. The term *"mucosal"* refers to a tissue comprising a mucous membranes, such as the nasal mucosa, pulmonary mucosa, oral mucosa (such as sublingual or buccal) or rectal mucosa. Compositions for administration through the mucosal route include for example nasal spray or nasal drops or aerosol for inhalation.

In the practice of the invention the amount of the compound incorporated in the pharmaceutical composition and the dosage may vary widely. Factors considered when determining the precise dosage are well known to those skilled in the art. Examples of such factors include, but are not limited to, age, sex and weight of the subject being treated, intended medical use of the compounds, severity of the disease, patient's general condition, the dosage form, route of administration being employed and the frequency with which the composition is to be administered.

Most preferably, the pharmaceutical composition is in the form of an oral preparation.

Because of their ease of administration, tablets and capsules are preferred and represent the most advantageous oral dosage unit form, in which case solid pharmaceutical excipients are employed. If desired, tablets may be coated by standard aqueous or non-aqueous techniques.

Preferably, the oral pharmaceutical compositions of the present invention may be administered in single or divided doses, from one to four times a day. The oral dosage -forms may be conveniently presented in unit dosage forms and prepared by any methods well known manner the art of pharmacy. Preferably, the therapeutically or prophylactically effective amount of an active ingredient ranges from about 20 mg to about 1000 mg daily (preferably administered orally), more preferably from about 50 mg to about 500 mg daily, and most preferably from about 100 mg to about 400 mg daily (preferably administered orally). The daily dose may be administered either singly or in multiple dosage over 24-hour period. For oral administration, the therapeutically effective amount of the active ingredient may be several times greater than that for, parenteral administration. The amount of the orally administered active ingredient may range from about five to ten times greater than that for intravenous or subcutaneous administration.

### Pharmaceutical Compositions and Dosage Forms Useful in the Invention

Pharmaceutical compositions and dosage forms which may be used in the invention comprise one or more of the active ingredients disclosed herein. Pharmaceutical compositions and dosage forms of the invention typically also comprise one or more pharmaceutically acceptable excipients or diluents (pharmaceutical acceptable carrier).

Single unit dosage forms of the invention are suitable for example for oral, mucosal (e.g., nasal, sublingual, buccal, pulmonary, or rectal mucosa), parenteral (e.g., subcutaneous, intravenous, bolus injection, intramuscular, intraarterial, intraperitoneal or subcutaneous), or transdermal administration to a patient.

Examples of dosage forms include, but are not limited to: tablets; caplets; capsules, such as soft elastic gelatin capsules; cachets; troches; lozenges; dispersions; suppositories ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions), emulsions (e.g., oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

### Oral Dosage Forms Pharmaceutical compositions

Oral dosage forms of the present invention suitable for oral administration may be presented as discrete pharmaceutical unit dosage forms, such as capsules, soft elastic gelatin capsules, tablets, caplets, cachets, or aerosols sprays, each containing a predetermined amount of the active ingredients, as a powder or granules, or as a solution or a suspension in an aqueous liquid, a non-aqueous liquid, an oil-in-water emulsion, or a water-in-oil liquid emulsion. Dosage forms such as oil-in-water emulsions typically comprise surfactants such as an anionic surfactant, for example anionic phosphate ester or lauryl sulfates, but other types of surfactants such as cationic or nonionic surfactants may be used in the compositions of the present invention. See generally, Remington's Pharmaceutical Sciences, 18th ed., Mack Publishing, Easton PA (1990).

Pharmaceutical compositions of the present invention suitable for oral administration may be formulated as a pharmaceutical composition in a soft elastic gelatin capsule unit dosage form by using conventional methods well known in the art. See, e.g., Ebert, Pharm. Tech, 1(5):44-50 (1977). Pharmaceutical compositions in the form of capsules or tablets coated by an entero-soluble gastroresistant film and which contains a lyophilisate consisting of glycosaminoglyean, a thickening agent, and a surfactant have been previously described in U.S. Patent No. 5,252,339. Soft elastic gelatin capsules have a soft, globular gelatin shell somewhat thicker than that of hard gelatin capsules, wherein a gelatin is plasticized by the addition of plasticizing agent, e.g., glycerin, sorbitol, or a similar polyol. The hardness of the capsule shell may be changed by varying the type of gelatin used and the amounts of plasticizer and water.

Typical oral dosage forms of the invention are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in liquid or aerosol dosage forms include, but are not limited to, water, glycols, oils, alcohols, flavoring agents, preservatives, and coloring agents. Examples of excipients suitable for use in solid oral dosage forms (e.g., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or non-aqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), microcrystalline cellulose, and mixtures thereof.

### Preparation of the compounds

The compounds of the present invention can be prepared according to the methods disclosed in Sterling et al (U.S. patent No. 5,880,157), M. Bialer et al, Pharm Res. 13:284-289 (1996) and Freifelder et al. J. Org. Chem. 26:203 (1961) or a modification thereof which will be apparent to those skilled in the art.

M-TMCD and TMCD were prepared according to the method disclosed in Sterling et al (U.S. patent No. 5,880,157).
Valnoctamide (VCD, powder) was given as a gift from Sanofi, France. 2,2,3,3-tetramethylcyclopropanecarboxylic acid (TMCA) can be obtained from Sigma Aldrich.

### BREIF DESCRIPTION OF THE DRAWING

**Fig 1**. Shows the threshold of mechanical sensitivity of the rat foot as a function of time after administration of M-TMCD, for five different concentrations of the drug (20, 40, 60, 80, 100 mg/kg) and for methyl cellulose control (4 ml/kg). The term "B. line" refers to baseline.
**Fig 2**. Shows the threshold of mechanical sensitivity of the rat foot as a function of time after administration of VCD, for five different concentrations of the drug (20, 40, 60, 80, 100 mg/kg) and for methylcellulose control (4 ml/kg). The term "B. line" refers to baseline.
**Fig 3**. Shows the threshold of mechanical sensitivity of the rat foot as a function of time after administration of TMCD, for four different concentrations of the drug (40, 80, 100, 150 mg/kg) and for methylcellulose control (4 ml/kg). The term "B. line" refers to baseline.
**Fig 4****.** Shows the threshold of mechanical sensitivity of the rat foot as a function of time after administration of VPA, for four different concentrations of the drug (200, 250, 300, 400 mg/kg) and for methylcellulose control (4 ml/kg). The term "B. line" refers to baseline.

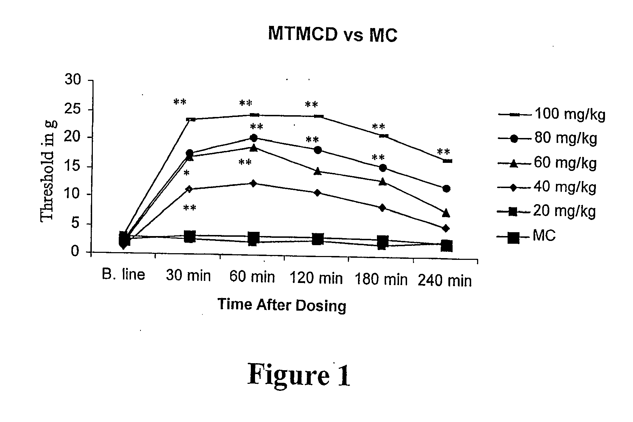

In figures 1-4: *, **, *** represent the statistical significance of the dose of the compound versus methylcellulose of the same experiment. '*' represent p<0.05, '**' represent p<0.01 and '***' represent p<0.001.
MC refers to methylcellulose.

### EXAMPLES

### 1. Experimental Procedures

### A. Neuropathic pain model

Male Sprague-Dawley rats (Harlan laboratories, Jerusalem, Israel) weighing 200 - 225 g were used throughout the study. The mechanical sensitivity (tactile allodynia) of the foot was quantified by the occurrence of foot withdrawal in response to normally innocuous mechanical stimuli using nine different von Frey filaments (VFF) ranging from 0.6 to 26 g. Rats that did not withdraw the foot to mechanical stimulus (von Frey filaments) of 15 g for 2 consecutive days (-2 and -1) before surgery were included in the study. At day 0, under xylazine-ketamine anesthesia, the L5 and L6 spinal nerves of one side of the rat were tightly ligated and cut in order to induce the development of neuropathic pain syndrome as disclosed in detail in Sheen, K. and Chung, J.M., Signs of neuropathic pain depend on signals from injured nerve fibers in a rat model, Brain Research 610: 62-68 (1993). The effect on neuropathic pain (tactile allodynia) was measured 5-6 days following operation as disclosed above. Rats responding to mechanical stimuli of 10g or less (in the operated leg) were eligible for the study and were included in the drug administration regime.

### B. Surgical procedure

The procedure of legating and cutting of the spinal nerves was performed as previously described by Sheen, K. and Chung, J.M., Signs of neuropathic pain depend on signals from injured nerve fibers in a rat model, Brain Research 610: 62-68 (1993). Briefly, under ketamine-xylazine anesthesia the rat was placed in a prone position and the left paraspinal muscles were separated from spinous processes at the L4-S2 level. Part of the L6 transverse process was removed and the L4-L6 spinal nerves were identified. The L5-L6 spinal nerves were isolated and tightly ligated and cut, distal to the dorsal root ganglion and proximal to the formation of the sciatic nerve. Following complete homeostasis the wound was sutured.

### C. Drug testing protocol

For all the studies, appropriate amounts of compounds were suspended in a solution of 0.5% (w/v) methylcellulose in double distilled water. The 0.5% w/v methyl cellulose vehicle was prepared by dissolving 1.25 g of methyl cellulose (viscosity of 2%, 4000 centipoises, Sigma) in 250 ml of double distilled water (DDW).

A volume of 4 ml/kg (volume of injected suspension/rat body weight) was then injected to the rats. The three drugs used were, M-TMCD and VCD at concentrations of 20, 40, 60, 80 and 100 mg/kg body weight, TMCD at concentrations of 40, 80, 100 and 150 mg/kg body weight, VPA at concentrations of 200, 250, 300, 400 mg/kg and methylcellulose was used as a control at 4 ml/kg (MC, vehicle). The three drugs and the control vehicle were administered i.p. (intraperitoneally) to rats at postoperative days 7, 14 and 21 in a double blind randomized crossover manner. Tactile allodynia (response to mechanical stimulus) was challenged using VFFs of 0.6 - 26 g at 30 min pre-dosing (Baseline (B. line)) and at 30, 60, 120, 180 and 240 min post-dosing. Rat that obtained threshold of at least 15 g, (back to pre-operation baseline threshold), was regarded as being free of tactile allodynia. This threshold was set for the purpose of ED₅₀ determination as the inclusion criteria to this study was 15 g (considered as the minimum non-allodynic threshold).

### D. Foot withdrawal measurements

Measurements of the foot withdrawal to normally innocuous mechanical stimuli were applied with set of 9 VFFs ranging from 0.6 g to 26 g. The rat was placed on a metal mesh floor covered with a transparent plastic dome, a period of acclimatization was allowed prior to testing. VFFs were applied from underneath the mesh floor to the plantar surface of the foot. Each trial consisted of repeated applications of each of the VFFs in an ascending order for 5 times, each for a period of 1 second. testing between two consecutive ascending VFFs was separated by period of 2 min. If the rat withdrew the foot at least 3 times out of 5 at a specific VFF no further ascending filaments were tested and this filament was considered as a withdrawal threshold (response). Mechanical stimulus trials with the series of ascending VFF were repeated 2 times for a given time point. The repeated measurements were averaged and taken as the paw withdrawal threshold on a given time point.

### E. Statistical analysis

The absolute threshold and the difference in the allodynic threshold (time point minus baseline) values were tested using Mann-Whitney Test and Kruskal-Wallis Nonparametric test (two tailed) followed by Dunn's Multiple comparison test. The ED₅₀ (median effective dose) was calculated by using Probit analysis method.

### Example 1: Decrease in neuropathic pain following administration of M-TMCD.

Male Sprague-Dawley rats (Harlan laboratories, Jerusalem, Israel) weighing 200 - 225 g were used. The rats chosen feature initial lack of foot withdrawal in response to mechanical stimuli, as described in experimental A (neuropathic pain model) above. The rats underwent surgery by legating and cutting of the L5-L6 nerves, as disclosed in Experimental B (surgical procedure) above , to produce neuropathic pain as verified by tactile allodynia. Rats were divided to 3 groups including 8-10 rats per study. Each group received 2 different doses of M-TMCD i.p (selected from 20,40, 60, 80 and 100 mg/kg) and 4 ml/kg of methyl cellulose was used as control. The doses of M-TMCD were 20,40, 60, 80 and 100 mg/kg.
Tactile allodynia threshold was measured as base line and 30,60, 120,180 and 240 minutes after administration of the drug, as described in the Experimental above, the threshold being the foot withdrawal in response to mechanical stimulus trials with the series of ascending VFF.
The results are shown in Fig 1. As can be seen, M-TMCD increased the threshold of tactile allodynia (proportional to the neuropathic pain) in a dose dependent manner, maximal increase in threshold, indicating maximal decrease in neuropathic pain was evident 60 min after administration of the drug with an ED₅₀ of 41 mg/kg.

### Example 2: Decrease in neuropathic pain following administration of VCD

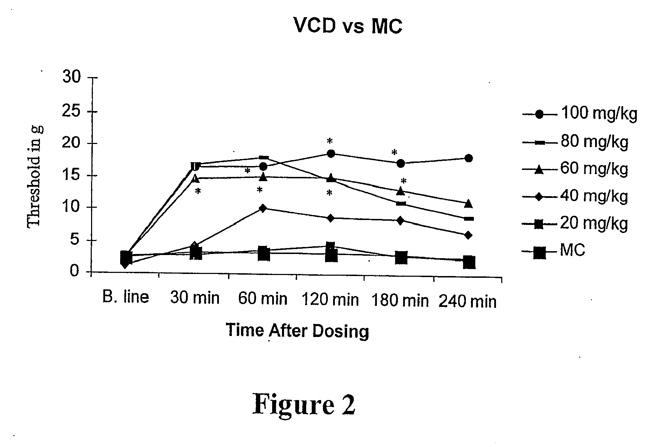

The same experiment described in Example 1 was repeated with VCD, and the results are shown in Fig 2.
As can be seen the rats responded in a dose dependent manner to varying dosages of VCD, with maximal effect evident 60 min after administration of the drug with an ED₅₀ of 52.3 mg/kg..

### Example 3: Decrease in neuropathic pain following administration of TMCD

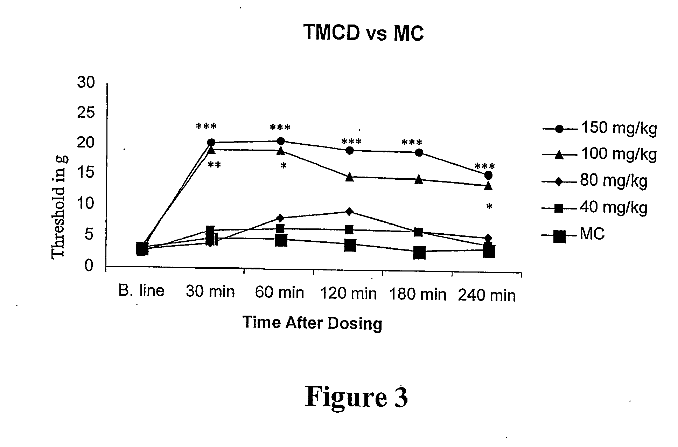

The same experiment described in Example 1 was repeated with TMCD, and the results are shown in Fig 3.
As can be seen the rats responded in a dose dependent manner to varying dosages of TMCD, with maximal effect evident 60 min after administration of the drug with an ED₅₀ of 84.5 mg/kg.

### Reference Example 4: Decrease in neuropathic pain following administration of valproic acid

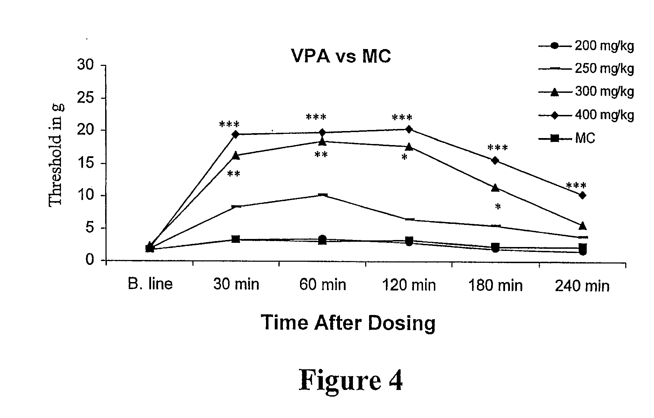

The same experiment described in Example 1 was repeated with VPA, and the results are shown in Fig 4.
As can be seen the rats responded in a dose dependent manner to varying dosages of VPA, with maximal effect evident 60 min after administration of the drug with an ED₅₀ of 269 mg/kg.

The results show that the compounds (TMCD, M-TMCD, VCD) are effective for treating neuropathic pain. The compounds were highly effective compared to valproic acid as evident by the lower ED₅₀ values (compared to valproic acid). The results show that M-TMCD and VCD are more preferred compounds and M-TMCD is the most preferred compound.

## Claims

1. A compound of formula I 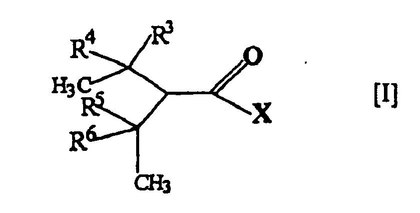 wherein
(i) X is selected from OH and NR¹R²;
(ii) R¹, R² are independently selected from H and C₁-C₆ alkyl; and
(iii)
(a) R³, R⁴ are independently selected from H and C₁-C₆ alkyl;
R⁵, R⁶ are independently selected from H and C₁-C₆ alkyl; or
(b) one of R³ and R⁴, together with one of R⁵ and R⁶, form a cyclopropyl ring, and the other of R³, R⁴, R⁵ and R⁶ is selected from H and C₁-Cₑ alkyl;
for the use in the treatment of a disease or condition selected from: neuropathic pain and migraine.

2. The compound of formula I according to claim 1 wherein said C₁-C₆ alkyl group is a straight or a branched alkyl group.

3. The compound of formula I according to claim 1 wherein the total number of carbon atoms of R³, R⁴, R⁵ and R⁶ in a compound of formula I is two.

4. The compound of formula I according to claim 1 wherein
(i) X is selected from OH and NR¹R²;
(ii) R¹, R² are independently selected from H and C₁-C₆ alkyl; and
(iii)
(a) R³, R⁴ are independently selected from H, methyl and ethyl;
R⁵, R⁶ are independently selected from H, methyl and ethyl; or
(b) one of R³ and R⁴, together with one of R⁵ and R⁶, form a cyclopropyl ring, and the other of R³, R⁴, R⁵ and R⁶ is selected from H, methyl and ethyl.

5. The compound of formula I according to claim 1 wherein X is selected from OH and NR¹R²; R¹, R² are independently selected from H and C₁-C₆ alkyl; and one of R³ and R⁴, together with one of R⁵ and R⁶ form a cyclopropyl ring, and the other of R³, R⁴, R⁵ and R⁶ is a methyl.

6. The compound of formula I according to claim 5 having the structural formula II 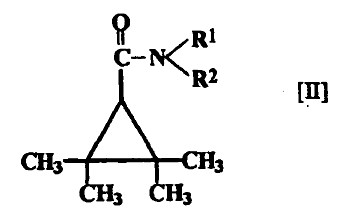 wherein R¹ and R² are independently selected from H and C₁-C₆ alkyl.

7. The compound of formula I according to claim 5 or 6 wherein at least one of R¹ and R² is H and the other of R¹ and R² is a C₁-C₆ alkyl.

8. The compound of formula I according to any one of claims 5-7 wherein said C₁-C₆ alkyl is a methyl.

9. The compound of formula I according to any one of claims 5-7 wherein said compound is N-methyl-2,2,3,3-tetramethylcyclopropanecarboxamide.

10. The compound of formula I according to claim 5 or 6 wherein said compound is 2,2,3,3-tetramethylcyclopropanecarboxamide.

11. The compound of formula I according to claim 1 wherein said compound is 2-ethyl-3-methyl-pentanoic acid amide.

12. The compound of formula I according to claim 1 wherein said compound is 2-ethyl-3-methyl-pentanoic acid.

13. The compound of formula I according to claim 5 wherein said compound is 2,2,3,3-tetramethylcyclopropanecarboxylic acid.

14. The compound of formula I according to claim 1 wherein said disease or condition is a neuropathic pain.

15. The compound of formula I according to claim 1 wherein the route of administration is selected from oral, parenteral, topical, transdermal, mucosal, rectal and buccal administration.

16. The compound of formula I according to claim 1 wherein the route of administration is selected from oral and parenteral administration.

17. The compound of formula I according to claim 15 or 16 wherein said parenteral route of administration is selected from intravenous, intramuscular, intraperitoneal and subcutaneous administration.

18. Use of a compound of formula I 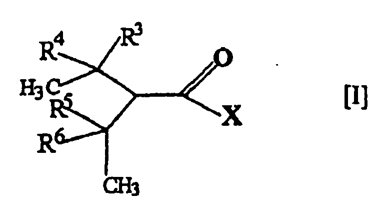 wherein
(i) X is selected from OH and NR¹R²;
(ii) R¹, R² are independently selected from H and C₁-C₆ alkyl; and
(iii)
(a) R³, R⁴ are independently selected from H and C₁-C₆ alkyl;
R⁵, R⁶ are independently selected from H and C₁-C₆ alkyl; or
(b) one of R³ and R⁴, together with one of R⁵ and R⁶, form a cyclopropyl ring, and the other of R³, R⁴, R⁵ and R⁶ is selected from H and C₁-C₆ alkyl;
for the preparation of a medicament for treating a disease or condition selected from: neuropathic pain and migraine.

## Patentansprüche

1. Verbindung der Formel I 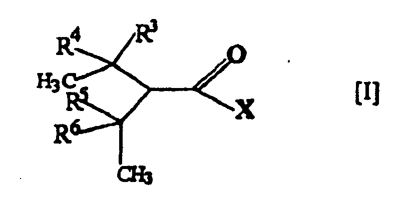 wobei
(i) X ausgewählt aus OH und NR¹R² ist;
(ii) R¹, R² unabhängig voneinander ausgewählt aus H und C₁-C₆ Alkyl sind; und
(iii)
(a) R³, R⁴ unabhängig voneinander ausgewählt aus H und C₁-C₆ Alkyl sind;
R⁵, R⁶ unabhängig voneinander ausgewählt aus H und C₁-C₆ Alkyl sind; oder
(b) eines von R³ und R⁴, zusammen mit einem von R⁵ und R⁶, einen Cyclopropylring bilden, und das andere von R³, R⁴, R⁵ und R⁶ ausgewählt aus H und C₁-C₆ Alkyl ist;
zur Verwendung in der Behandlung von einer Krankheit oder einem Zustand ausgewählt aus: neuropathischer Schmerz und Migräne.

2. Verbindung der Formel I nach Anspruch 1, wobei die genannte C₁-C₆ Alkylgruppe eine geradkettige oder verzweigte Alkylgruppe ist.

3. Verbindung der Formel I nach Anspruch 1, wobei die Gesamtanzahl von Kohlenstoffatomen von R³, R⁴, R⁵ und R⁶ in einer Verbindung der Formel I zwei ist.

4. Verbindung der Formel I nach Anspruch 1, wobei
(i) X ausgewählt aus OH und NR¹R² ist;
(ii) R¹, R² unabhängig voneinander ausgewählt aus H und C₁-C₆ Alkyl sind; und
(iii)
(a) R³, R⁴ unabhängig voneinander ausgewählt aus H, Methyl und Ethyl sind;
R⁵, R⁶ unabhängig voneinander ausgewählt aus H, Methyl und Ethyl sind; oder
(b) eines von R³ und R⁴, zusammen mit einem von R⁵ und R⁶, einen Cyclopropylring bilden, und das andere von R³, R⁴, R⁵ und R⁶ ausgewählt aus H, Methyl und Ethyl ist.

5. Verbindung der Formel I nach Anspruch 1, wobei X ausgewählt aus OH und NR¹R² ist; R¹,R² unabhängig voneinander ausgewählt aus H und C₁-C₆ Alkyl sind; und eines von R³ und R⁴, zusammen mit einem von R⁵ und R⁶, einen Cyclopropylring bilden, und das andere von R³, R⁴, R⁵ und R⁶ ein Methyl ist.

6. Verbindung der Formel I nach Anspruch 5, die die Strukturformel II besitzt 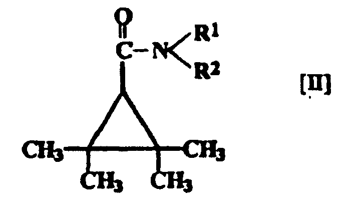 wobei R¹ und R² unabhängig voneinander ausgewählt aus H und C₁-C₆ Alkyl sind.

7. Verbindung der Formel I nach Ansprüchen 5 oder 6, wobei mindestens eines von R¹ und R² H ist und das andere von R¹ und R² ein C₁-C₆ Alkyl ist.

8. Verbindung der Formel I nach einem der Ansprüche 5 bis 7, wobei das genannte C₁-C₆ Alkyl ein Methyl ist.

9. Verbindung der Formel I nach einem der Ansprüche 5 bis 7, wobei die genannte Verbindung N-Methyl-2,2,3,3-tetramethylcyclopropancarboxamid ist.

10. Verbindung der Formel I nach Anspruch 5 oder 6, wobei die genannte Verbindung 2,2,3,3-Tetramethylcyclopropancarboxamid ist.

11. Verbindung der Formel I nach Anspruch 1, wobei die genannte Verbindung 2-Ethyl-3-methyl-pentansäureamid ist.

12. Verbindung der Formel I nach Anspruch 1, wobei die genannte Verbindung 2-Ethyl-3-methyl-pentansäure ist.

13. Verbindung der Formel I nach Anspruch 5, wobei die genannte Verbindung 2,2,3,3-Tetramethylcyclopropancarbonsäure ist.

14. Verbindung der Formel I nach Anspruch 1, wobei die genannte Krankheit oder der Zustand ein neuropathischer Schmerz ist.

15. Verbindung der Formel I nach Anspruch 1, wobei die Applikationsform ausgewählt ist aus oraler, parenteraler, topikaler, transdermaler, mukosaler, rektaler und bukkaler Applikation.

16. Verbindung der Formel I nach Anspruch 1, wobei die Applikationsform ausgewählt ist aus oraler und parenteraler Applikation.

17. Verbindung der Formel I nach Anspruch 15 oder 16, wobei die genannte parenterale Applikationsform ausgewählt ist aus intravenöser, intramuskularer, intraperitonealer und subkutaner Applikation.

18. Verwendung einer Verbindung der Formel I 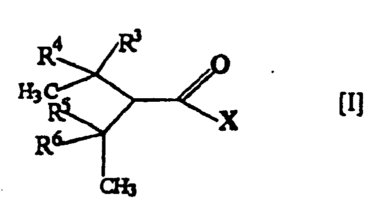
(i) X ausgewählt aus OH und NR¹R² ist;
(ii) R¹, R² unabhängig voneinander ausgewählt aus H und C₁-C₆ Alkyl sind; und
(iii)
(a) R³, R⁴ unabhängig voneinander ausgewählt aus H und C₁-C₆ Alkyl sind;
R⁵, R⁶ unabhängig voneinander ausgewählt aus H und C₁-C₆ Alkyl sind; oder
(b) eines von R³ und R⁴ zusammen mit einem von R⁵ und R⁶ einen Cyclopropylring bilden, und das andere von R³, R⁴, R⁵ und R⁶ ausgewählt aus H und C₁-C₆ Alkyl ist;
zur Herstellung eines Medikaments zum Behandeln einer Krankheit oder eines Zustands ausgewählt aus: neuropathischer Schmerz und Migräne.

## Revendications

1. Composé de formule I 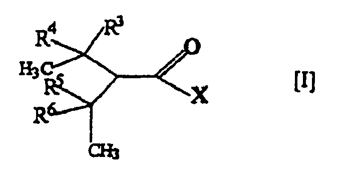 dans laquelle
(i) X est choisi parmi OH et NR¹R²;
(ii) R¹, R² sont indépendamment choisis parmi H et un alkyle en C₁-C₆ ; et
(iii)
(a) R³, R⁴ sont indépendamment choisis parmi H et un alkyle en C₁-C₆ ;
R⁵, R⁶ sont indépendamment choisis parmi H et un alkyle en C₁-C₆ ; ou
(b) l'un parmi R³ et R⁴, conjointement avec l'un parmi R⁵ et R⁶, forment un noyau cyclopropyle, et l'autre parmi R³, R⁴, R⁵ et R⁶ est choisi parmi H et un alkyle en C₁-C₆ ;
pour l'utilisation dans le traitement d'une maladie ou d'une affection choisie parmi : une douleur neuropathique et une migraine.

2. Composé de formule I selon la revendication 1, dans lequel ledit groupe alkyle en C₁-C₆ est un groupe alkyle linéaire ou ramifié.

3. Composé de formule I selon la revendication 1, dans lequel le nombre total d'atomes de carbone de R³, R⁴, R⁵ et R⁶ dans un composé de formule I est de deux.

4. Composé de formule I selon la revendication 1, dans lequel
(i) X est choisi parmi OH et NR¹R² ;
(ii) R¹, R² sont indépendamment choisis parmi H et un alkyle en C₁-C₆ ; et
(iii)
(a) R³, R⁴ sont indépendamment choisis parmi H, méthyle et éthyle ;
R⁵, R⁶ sont indépendamment choisis parmi H, méthyle et éthyle ; ou
(b) l'un parmi R³ et R⁴, conjointement avec l'un parmi R⁵ et R⁶, forment un noyau cyclopropyle, et l'autre parmi R³, R⁴, R⁵ et R⁶ est choisi parmi H, méthyle et éthyle.

5. Composé de formule I selon la revendication 1, dans lequel X est choisi parmi OH et NR¹R² ; R¹, R² sont indépendamment choisis parmi H et un alkyle en C₁-C₆ ; et l'un parmi R³ et R⁴, conjointement avec l'un parmi R⁵ et R⁶, forment un noyau cyclopropyle, et l'autre parmi R³ R⁴, R⁵ et R⁶ est un méthyle.

6. Composé de formule I selon la revendication 5 ayant la formule structurale II 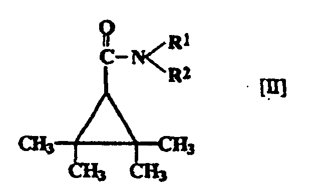 dans laquelle R¹ et R² sont indépendamment choisis parmi H et un alkyle en C₁-C₆.

7. Composé de formule I selon la revendication 5 ou 6, dans lequel au moins l'un parmi R¹ et R² est H et l'autre parmi R¹ et R² est un alkyle en C₁-C₆.

8. Composé de formule I selon l'une quelconque des revendications 5 à 7, dans lequel ledit alkyle en C₁-C₆ est un méthyle.

9. Composé de formule I selon l'une quelconque des revendications 5 à 7, dans lequel ledit composé est le N-méthyl-2,2,3,3-tétraméthylcyclopropanecarboxamide.

10. Composé de formule I selon la revendication 5 ou 6, dans lequel ledit composé est le 2,2,3,3-tétraméthylcyclopropanecarboxamide.

11. Composé de formule I selon la revendication 1, dans lequel ledit composé est un amide de l'acide 2-éthyl-3-méthyl-pentanoïque.

12. Composé de formule I selon la revendication 1, dans lequel ledit composé est l'acide 2-éthyl-3-méthyl-pentanoïque.

13. Composé de formule I selon la revendication 5, dans lequel ledit composé est l'acide 2,2,3,3-tétraméthylcyclopropanecarboxylique.

14. Composé de formule I selon la revendication 1, dans lequel ladite maladie ou affection est une douleur neuropathique.

15. Composé de formule I selon la revendication 1, dans lequel la voie d'administration est choisie parmi l'administration orale, parentérale, topique, transdermique, muqueuse, rectale et buccale.

16. Composé de formule I selon la revendication 1, dans lequel la voie d'administration est choisie parmi l'administration orale et parentérale.

17. Composé de formule I selon la revendication 15 ou 16, dans lequel ladite voie d'administration parentérale est choisie parmi l'administration intraveineuse, intramusculaire, intrapéritonéale et sous-cutanée.

18. Utilisation d'un composé de formule I 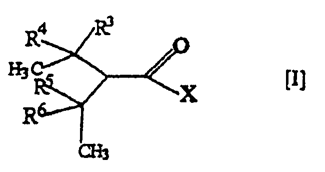 dans laquelle
(i) X est choisi parmi OH et NR¹R² ;
(ii) R¹, R² sont indépendamment choisis parmi H et un alkyle en C₁-C₆ ; et
(iii)
(a) R³, R⁴ sont indépendamment choisis parmi H et un alkyle en C₁-C₆ ;
R⁵, R⁶ sont indépendamment choisis parmi H et un alkyle en C₁-C₆ ; ou
(b) l'un parmi R³ et R⁴, conjointement avec l'un parmi R⁵ et R⁶, forment un noyau cyclopropyle, et l'autre parmi R³, R⁴, R⁵ et R⁶ est choisi parmi H et un alkyle en C₁-C₆ ;
pour la préparation d'un médicament pour traiter une maladie ou une affection choisie parmi : une douleur neuropathique et une migraine.
